# EUROPEAN PATENT APPLICATION

(11) **EP 1 195 437 A1**
(43) Date of publication of application: **10.04.2002**
(21) Application number: 01308502.2
(22) Date of filing: 04.10.2001
(51) Int. Cl.: C12N 15/72, C12N 5/10

(54) **Process for the recombinant production of proteins using a Plac/Np hybrid promoter**

(30) Priority: 04.10.2000 JP 2000305132
(71) Applicant: MITSUI CHEMICALS, INC., Tokyo (JP)
(72) Inventor: Naitou, Naokazu, Mitsui Chemicals, Inc., Mobara-shi, Chiba (JP); Matsumoto, Kazuya, c/o Mitsui Chemicals, Inc., Mobara-shi, Chiba (JP); Higashimura, Norikazu. c/o Mitsui Chem. Inc., Mobara-shi, Chiba (JP); Tsunekawa, Bunkichi, c/o Mitsui Chem. Inc., Tokyo (JP); Uchida, Hiroshi, c/o Mitsui Chemicals, Inc., Mobara-shi, Chiba (JP); Wada, Mitsufumi, c/o Mitsui Chemicals, Inc., Mobara-shi, Chiba (JP)
(74) Representative: Stuart, Ian Alexander

(57) **Abstract**

An objective of this invention is to provide an effective process for producing a recombinant protein using E. coli and a technique therefor.

A DNA cassette comprising the first promoter DNA derived from a lac promoter in a pUC 19 plasmid, a spacer region DNA and the second promoter DNA derived from a promoter region in a neutral protease in Bacillus amyloliquefaciens wherein these are sequentially aligned in series in a 5' to 3' direction is prepared and is used as a promoter for expressing a recombinant protein in E. coli.

## Description

This invention relates to a DNA cassette for expressing a protein acting as a promoter; a recombinant plasmid in which a coding region encoding a protein is combined with the DNA cassette; an E. coli transformant transformed by the recombinant plasmid; and a process for producing a protein using the E. coli transformant, for allowing higher productivity to be achieved in protein production using E. coli.

Recent advance in gene recombination technique has provided recombinant proteins using a microorganism such as E. coli as a host, among which, as a medicine, human growth hormone, granulocyte colony stimulating factor (G-CSF) and so on have been used in a variety of applications. Production processes of a protein using E. coli as a host can be classified into two groups: intracellular processes in which a desired protein is stored in a cell and secretion processes in which a desired protein is stored in a periplasm which is a space between an inner and an outer membranes.

The latter secretion process has an advantage that a methionine residue is not contained in an N-terminus in a protein and a natural type protein whose higher-order structure comprises an active structure. In the process, two processes of processing and inner-membrane passage are, however, required for protein secretion so that generally they cannot give high productivity. Furthermore, the size of a protein which can be produced by secretion is limited. Therefore, this type of process may not be necessarily suitable to industrial production.

On the other hand, in an intracellular process, a methionine residue generally remains in an N-terminus in an expressed protein and there are some cases where its higher-order structure may not be of an active type. In such a case, production of a natural type protein requires removal of the methionine in the N-terminus and unfolding/refolding of the protein. An intracellular process has advantageous characteristics that it can provide a significantly higher production amount than a secretion process and it may permit expression of a protein with a large molecular weight which cannot be achieved by a secretion process to be produced.

A protein expressed by an intracellular process is often produced as an inclusion body in a cell. An intracellular inclusion body thus formed can be recovered with a purity of 90 % or higher only by washing, by centrifugation, a disrupted cell suspension after a cell disruption treatment. It is advantageous to obtain a desired protein with a reduced amount of contaminating proteins for facilitating a subsequent purification procedure.

It is, however, known that even such an intracellular process having an advantage of a higher expression amount cannot provide an adequate production amount, depending on the type of a protein to be produced. In particular, it is generally known that a protein derived from a mammal is obtained in a less amount than a protein derived from a procaryote such as E. coli. Generally, in an intracellular process using E. coli, a protein derived from a procaryote may give a higher expression rate of 40 % or more of the total proteins in E. coli used, while, for example, human granulocyte colony stimulating factor (hG-CSF) gives at most about 10 %, specifically 3 to 5 % in US Patent No. 5676941 and about 10 % in Jpn. J. Cancer Res., 78, p. 1179-1181 (1987) and human fibroblast growth factor (hFGF-2) gives about 10 %, specifically 10 % in JP-A 5-508075 and 8 % in Journal of Biotechnology, 22, p. 299-310 (1992). Thus, in expressing a protein derived from a mammal, productivity is relatively lower than a protein derived from a procaryote.

In an intracellular production in E. coli, there have known several factors which may influence an expression amount. Among them, an expression promoter is an important factor. Many promoters such as a trp promoter and a pL promoter are known, but it has been needed to develop a promoter whereby a further improved production efficiency can be achieved.

### SUMMARY OF THE INVENTION

A preferred embodiment of this invention may provide a DNA cassette exhibiting promoter ability whereby higher productivity can be achieved in production of a protein derived from a mammal in E. coli, in particular an intracellular production process.

Preferred embodiments of other aspects of this invention may provide a plasmid useful in producing a protein comprising such a DNA cassette exhibiting promoter ability; a recombinant plasmid for protein expression in which a DNA encoding a desired protein is contained at a given position in the plasmid, an E. coli transformed with the recombinant plasmid; and a process for producing a protein using the E. coli.

The present inventors have intensely conducted investigation, focusing a promoter for achieving higher productivity in production of proteins derived from a variety of mammals when using an intracellular production process. A pUC 19 plasmid is a readily commercially available plasmid for E. coli. It is widely known that a lac promoter present in the plasmid is a potent promoter having a property that a inducing agent such as IPTG can be added to induce expression of a desired protein. However, when this promoter was applied to an hG-CSF expression system, a lower production amount as in the prior art described above was given, i.e., only 10 % of the total cell proteins (See Comparative Example 1).

On the other hand, a promoter involved in expression of a neutral protease in Bacillus amyloliquefaciense (Np promoter) is a promoter which has exhibited higher secretion productivity in secretion expression of, for example, human growth hormone in E. coli (for example, JP-B 8-24586). However, when the promoter was applied to a hG-CSF expression system, a lower production amount as in the prior art described above was given, i.e., only 10 % of the total cell proteins (See Comparative Example 1).

These results implicate that an existing promoter cannot be used for significantly improving an expression level in the prior art (about 10 % of the total cell proteins) for a protein derived from a mammal.

In view of the technical level in the prior art, the present inventors have intensely attempted to find a DNA exhibiting potent and universal promoter ability. Finally, they have found that an expression level considerably higher than those previously reported can be achieved, specifically an extremely higher level of 50 % or more of the total cell proteins can be achieved by using E. coli transformed by a recombinant plasmid where a DNA encoding a desired protein, in particular a protein derived from a mammal, is functionally ligated to a DNA cassette for protein expression in which a DNA derived from a lac promoter region and functioning as a promoter and a DNA derived from an Np promoter region and functioning as a promoter are aligned in series via a spacer region DNA in a 5' to 3' direction. This invention is based on our findings described above.

Specifically, a DNA cassette of this invention is a DNA cassette functioning as a promoter for protein expression in E. coli, comprising the first promoter derived from a lac promoter in a pUC 19 plasmid, a spacer region and the second promoter derived from a promoter region in a neutral protease in Bacillus amyloliquefaciense wherein the first promoter, the spacer region and the second promoter are sequentially aligned in series in a 5' to 3' direction.

It has been known that a plurality of promoters may be aligned in series to constitute a more potent promoter. It is crucial in this invention that a lac promoter and an Np promoter are aligned from 5' to 3'. We extensively investigated a DNA cassette having another combination, i.e., a cassette in which a lac and a lac promoters, an Np and a lac promoters or an Np and an Np promoters were sequentially aligned in series from 5' to 3'. In any cassette, productivity was significantly lower than that achieved using, as a promoter, a DNA cassette consisting of a lac and an Np promoter according to this invention.

A plasmid useful for forming a recombinant plasmid for protein expression in E. coli according to this invention is characterized in that it comprises the DNA cassette having the above structure and a region permitting replication in E. coli.

A recombinant plasmid for protein expression in E. coli according to this invention is characterized in that it comprises a DNA cassette exhibiting promoter ability; a coding region encoding a desired protein which is functionally ligated to the 3' end in the DNA cassette; and a region for replication in E. coli, wherein the promoter is comprised of the DNA cassette with the above structure.

A recombinant E. coli useful in a process for producing a protein according to this invention is characterized that it can be transformed with the recombinant plasmid with the above structure and can produce the protein encoded in the coding region in the recombinant plasmid.

A process for producing a protein according to this invention is characterized in that using E. coli comprising the steps of culturing the recombinant E. coli with the above structure to produce the protein encoded in the coding region in the recombinant plasmid carried by the recombinant E. coli, which is acclumulated within the cells of the recombinant E. coli; and recovering the protein from the cells.

This invention allows us to produce a protein derived from a mammal, in particular human granulocyte colony stimulating factor (hG-CSF), human fibroblast growth factor (hFGF-2) or canine growth hormone in a higher production amount which cannot be achieved using E. coli as a host according to the prior art. Thus, the prior art cannot achieve the level attainable by this invention.

### BRIEF DESCRIPTION OF THE DRAWINGS

Fig. 1 shows construction of an hG-CSF expression plasmid comprising an Np promoter for expressing hG-CSF in a pBR322 plasmid (pKK-mGCSF).
Fig. 2 shows construction of an hG-CSF expression plasmid comprising a cassette for protein expression according to this invention in a pUC19 plasmid (pGCSF6).
Fig. 3 shows construction of an hG-CSF expression plasmid comprising only a lac promoter for expressing hG-CSF in a pUC19 plasmid (pLAC-mGCSF).
Fig. 4 shows construction of an hG-CSF expression plasmid comprising only an Np promoter for expressing hG-CSF in a pUC19 plasmid (pNpr-mGCSF).
Fig. 5 shows construction of an hFGF-2 expression plasmid comprising an Np promoter for expressing hFGF-2 in a pBR322 plasmid (pKK-mFGF).
Fig. 6 shows construction of an hFGF-2 expression plasmid comprising a cassette for protein expression according to this invention for expressing hFGF-2 in a pUC19 plasmid (pFGF6).
Fig. 7 shows construction of a canine GH expression plasmid comprising only an Np promoter for expressing canine GH in a pBR322 plasmid (pKK-mcGH).
Fig. 8 shows construction of a canine GH expression plasmid comprising a cassette for protein expression according to this invention for expressing canine GH in a pBR322 plasmid (pcGH6).

### DETAILED DESCRIPTION OF PREFERRED EMBODIMENTS

This invention will be described in detail. A DNA cassette for protein expression according to this invention has a structure that the first promoter derived from a lac promoter region in a pUC 19 plasmid and the second promoter derived from a promoter (Np promoter) region in a neutral protease in Bacillus amyloliquefaciense are linked together by series via a spacer region in a 5' to 3' direction. The DNA cassette is attached to the 3' end such that a DNA (gene) encoding a desired protein is functionally ligated, i.e., such that expression of a gene for the desired protein is directed by the DNA cassette. When it is used for constituting a recombinant plasmid in combination with a region which permits replication in E. coli, it may allow the desired protein to be expressed in E. coli.

It is well known that a DNA in a lac promoter in a pUC19 plasmid and an Np promoter has potent promoter activity. It cannot be speculated from the existing data that among many promoters, these two promoters are selected in combination and a DNA cassette in which a lac promoter, a spacer region and an Np promoter are sequentially linked in series may improve a production efficiency by 5 folds or more in comparison with using an individual promoter.

A pUC19 plasmid may be that available from a commercial supplier such as Pharmacia Biotech. A lac promoter region as used herein refers to 470th to 682th bases in 2686 bases of the pUC19 plasmid sequence.

In this invention, the entire sequence of this 470th to 682th bases (SEQ ID NO: 1) or a partial sequence exhibiting promoter activity obtained from the entire sequence may be used as the first promoter derived from a lac promoter region. For example, a sequence in which 47 bases in the 5' side are deleted exhibits desired promoter activity and thus can be used in constituting a DNA cassette of this invention. Furthermore, a sequence of the first promoter derived from a lac promoter region exhibiting promoter activity may comprise deletion, substitution or insertion of one or two bases as long as the promoter activity can be maintained. Also may be used a sequence having a change such as deletion, substitution and insertion of one or more bases such that it can be hybridized with a sequence complementary to the promoter sequence under stringent conditions.

A spacer region linked with the 3' end in the first promoter region is crucial for proper action of the DNA cassette of this invention. It may have a length of 10 to 100 bp, preferably 15 to 70 bp, more preferably 20 to 60 bp, further preferably 25 to 45 bp. There are no specific restrictions to its sequence as long as it may give promoter activity desired in this invention, but it is convenient to use the partial sequence of the 5' end in the structural gene of the lacZ protein present in the 3' side of the lac promoter in the pUC19 plasmid as it is. In this case, the amino-acid partial sequence of the N-terminus in the lacZ protein is expressed in E. coli, but according to our investigation, it has been confirmed that presence of expression of the lacZ protein does not influence the expression amount of the desired protein. When there is a concern that contamination with an expressed lacZ protein may affect a purification operation for the desired protein, a codon encoding the methionine in the N-terminus of the lacZ protein in the part used in the spacer region from the lacZ gene may be modified into an appropriate codon to prevent a protein derived from the lacZ gene from being expressed.

An Np promoter is a promoter involved in secretion of a neutral protease of Bacillus amylolquefaciens. Since it functions in E. coli, it has been applied to secretion production of a human growth hormone (US Patent No. 5759810). The Np promoter has the sequence of SEQ ID NO: 3 (US Patent No. 5015574). The second promoter derived from the Np promoter of this invention may be that having the sequence of SEQ ID NO: 3 or a partial sequence exhibiting promoter activity which may be obtained from the above sequence. An example of the partial sequence is that of SEQ ID NO: 2, i.e., that of SEQ ID NO: 3 in which a partial sequence at the 5' end is deleted.

Furthermore, a sequence comprising the second promoter derived from the Np promoter may be that having a change such as deletion, substitution and insertion of one or two bases. Also may be used a sequence having a change such as deletion, substitution and insertion of one or more bases such that it can be hybridized with a sequence complementary to the promoter sequence under stringent conditions.

The Np promoter has been disclosed in the above UP Patent (US Patent No. 5759810). It may be obtained from a plasmid pGHR10 in an E. coli strain MT-10765. The MT-10765 strain has been deposited in National Institute of Bioscience and Human-Technology, Agency of Industrial Science and Technology, 1-1-3 Higashi, Tsukuba, Ibaragi as an international deposition according to Budapest Treaty under the accession No. FERM BP-5020.

A DNA (gene) encoding a desired protein to be expressed may be ligated to a DNA cassette for protein expression according to this invention to form a coding region, which may be then incorporated into a plasmid together with a region for replication in E. coli to provide a recombinant plasmid.

In addition, a DNA cassette for protein expression according to this invention may be incorporated into a plasmid together with a region for replication in E. coli to form a plasmid retainable or replicable in E. coli. The plasmid may be utilized as a material for preparing a recombinant plasmid for protein expression. An appropriate spacer may be, if desired, located at a given position, i.e., a position at which a DNA cassette make a command for expression of the plasmid to insert a DNA encoding a desired protein for forming a coding region and finally for providing a recombinant plasmid for expressing the desired protein.

There are no restrictions to a target protein to be expressed in E. coli utilizing promoter ability of a DNA cassette in a recombinant plasmid as long as E. coli can be expressed by promoter activity of the DNA cassette. The DNA cassette according to this invention is effective in improving the expression amount of a protein derived from a mammal. Among others, it is particularly effective in improving the expression amount in hG-CSF, hFGF-2 or canine growth hormone.

As used herein, the term " a protein derived from a mammal" refers to a protein produced by a mammal, but it includes a natural protein derived from a mammal which has been subject to an artificial modification.

It is known that when a gene sequence (codon) in a mammal body is used as a DNA (gene) encoding a desired protein which is ligated to a 3' side in a DNA cassette for protein expression as it is, there are some cases where its expression may be difficult due to a sequence in its 5' end. It is, therefore, preferable to modify 10 to 20 bps in the 5' end in the gene of the desired protein into a codon preferred by E. coli without changing the amino acid sequence as described later in Examples 1 to 3.

A region allowing replication in E. coli may be a DNA region comprising a replication origin functioning in E. coli. For example, a region for replication in a plasmid which has provided good results and has been demonstrated to be safe such as pUC19 and pBR322 may be preferably used.

If necessary, a drug resistance marker gene or a terminator may be incorporated in a recombinant plasmid according to this invention.

E. coli may be transformed with a recombinant plasmid according to this invention to provide a recombinant E. coli. Such transformation may be conducted as usual, for example, by a calcium chloride method. A host E. coli for transformation may be one without pathogenicity and frequently used; examples of a suitable strain include JM109, HB101 and W3110 (ATCC 27325), wherein an ATCC number refers to a number for a microorganism retained by American Type Culture Collection and ATCC 27325 may be available for value for any person as requested.

In producing a desired protein, a recombinant E. coli may be cultured using, for example, a culture medium containing a carbon source, a nitrogen source and inorganic salts which the recombinant E. coli can assimilate; preferable examples include an LB medium (10 g/L polypeptone and 5 g/L yeast extract). Glycelol as a carbon source may be added at a concentration of 0.2 to 1.5 wt% to further improve productivity. Culturing is conducted at 30 to 40 °C, preferably 37 °C while aerating the system for 16 to 24 hours.

In a recombinant E. coli of this invention using the DNA cassette having the above structure, the need for adding an inducer such as IPTG can be eliminated and a desired protein may be gradually accumulated within cells as culturing proceeds.

The desired protein may be recovered from the cultured cells by collecting the cells from the culture medium by centrifugation, suspending the cells in an appropriate buffer, physically disrupting the cells using, e.g., a French press and treating the disrupted cell suspension by, for example, centrifugation to recover the desired protein contained in the disrupted cell suspension. When the desired protein is stored as an inclusion body in a cell, the protein may be recovered in a precipitate obtained by centrifugation of a disrupted cell suspension. When it is stored as a soluble protein, it may be recovered in a supernatant obtained by cell disruption. For example, in examples described later, hG-CSF and canine growth hormone (canine GH) stored in cells as an inclusion body were recovered as a precipitate while hFGF-2 stored as a soluble protein in cells was recovered in a supernatant.

An inclusion body recovered as a precipitate may be, for example, reduced in a reduction solution prepared by adding a reducing agent such as 2-mercaptoethanol or DTT to a modifier such as 8M urea and 6M guanidine and then diluting the mixture in a solution containing an oxidizing agent such as glutathione to conduct unfolding/refolding of the desired protein recovered. The desired protein thus solubilized may be purified using a purification process such as ion exchange, gel filtration and affinity column chromatography or, if necessary, a combination thereof.

There has been described an application of a DNA cassette according to this invention to an intracellular expression process, but a DNA cassette according to this invention might be used as a promoter for a strain capable of secretion-production to significantly improve an efficiency in secretion production in a periplasm in E. coli.

### Examples

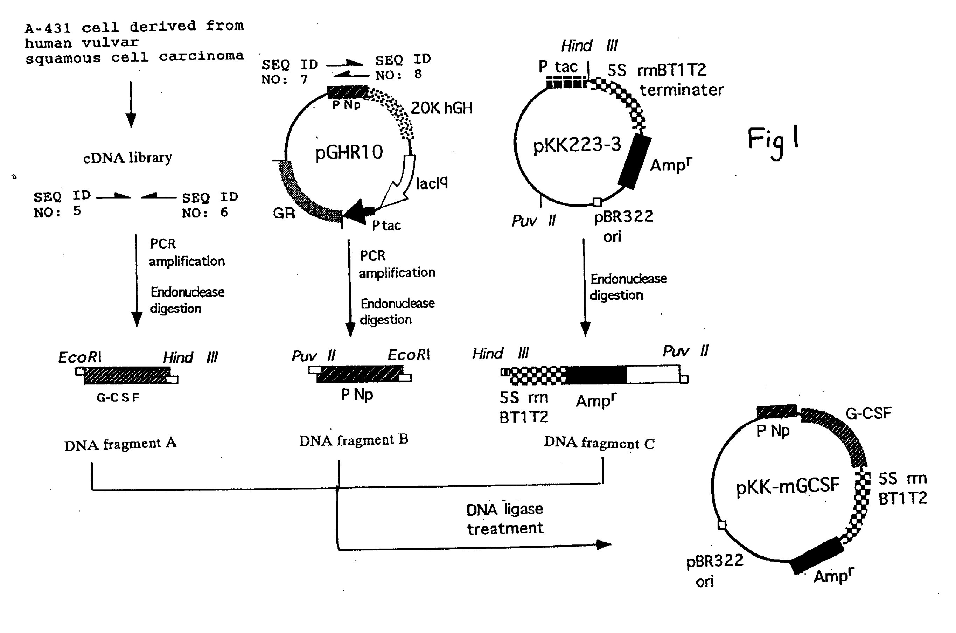

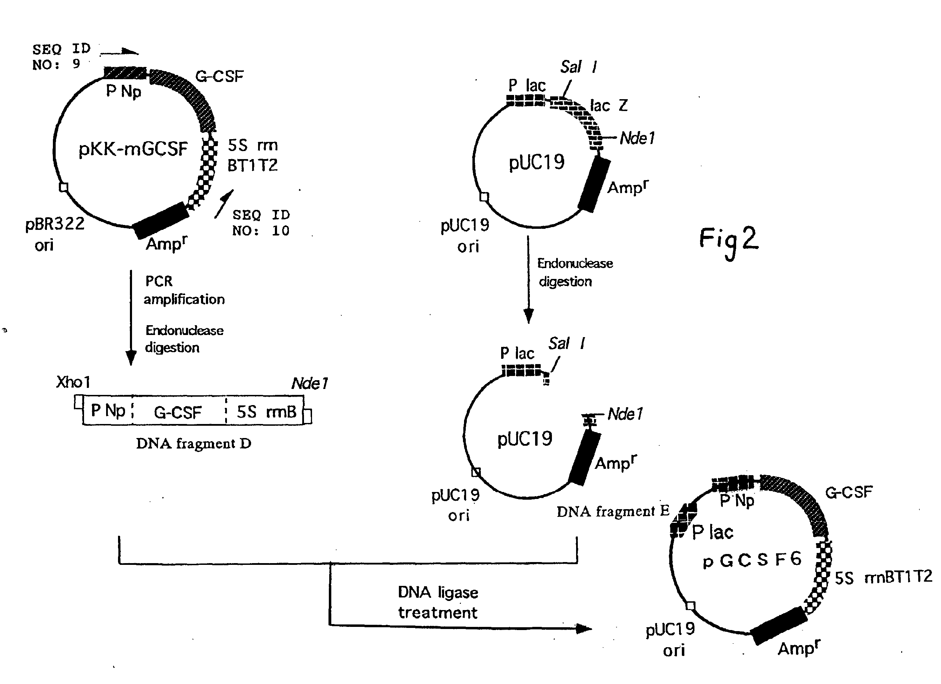

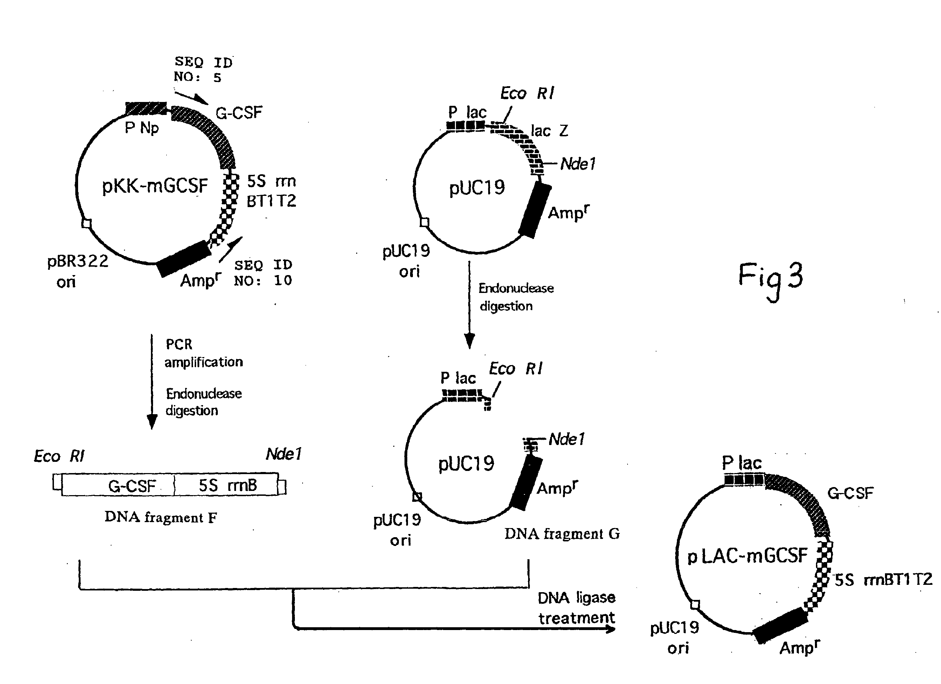

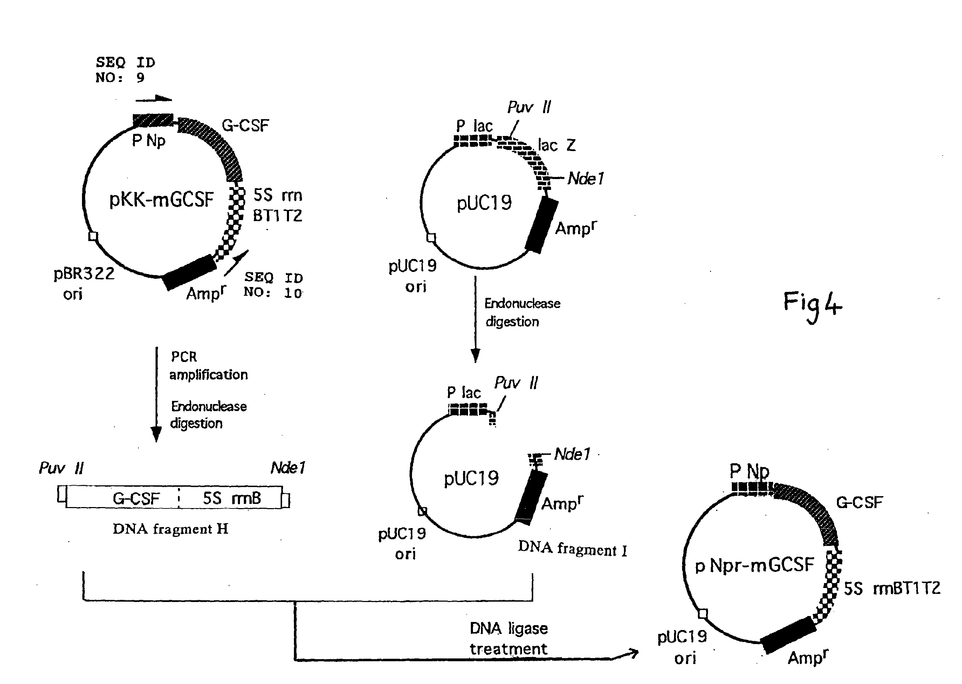

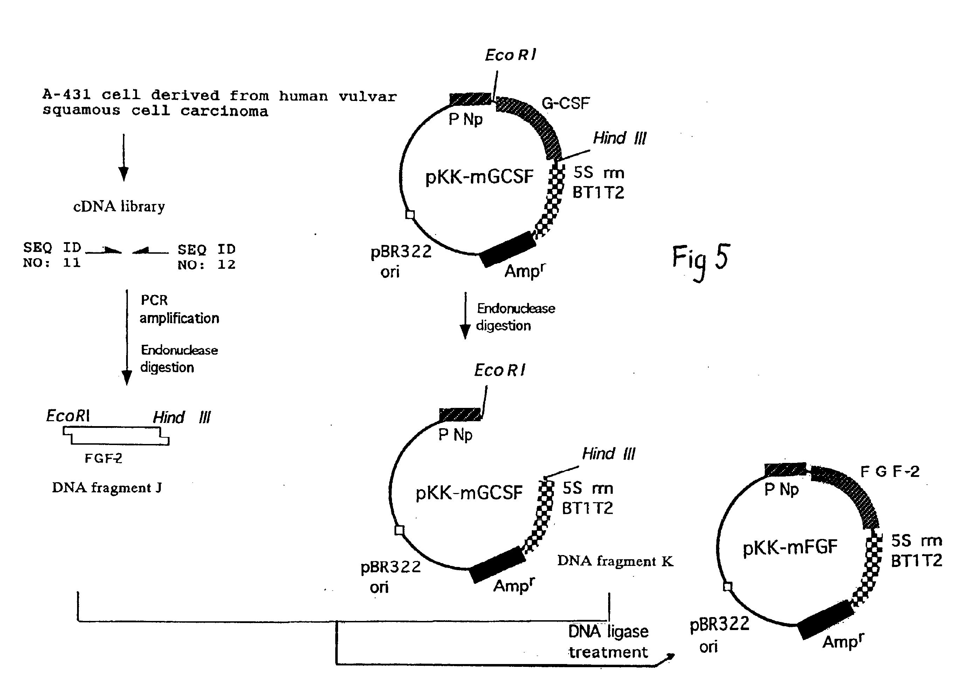

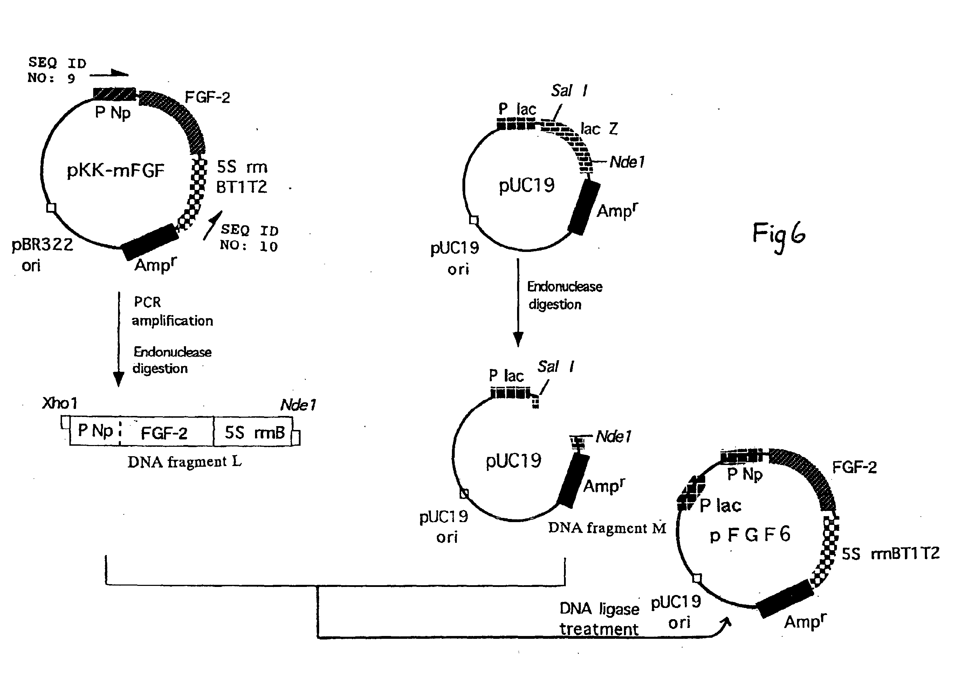

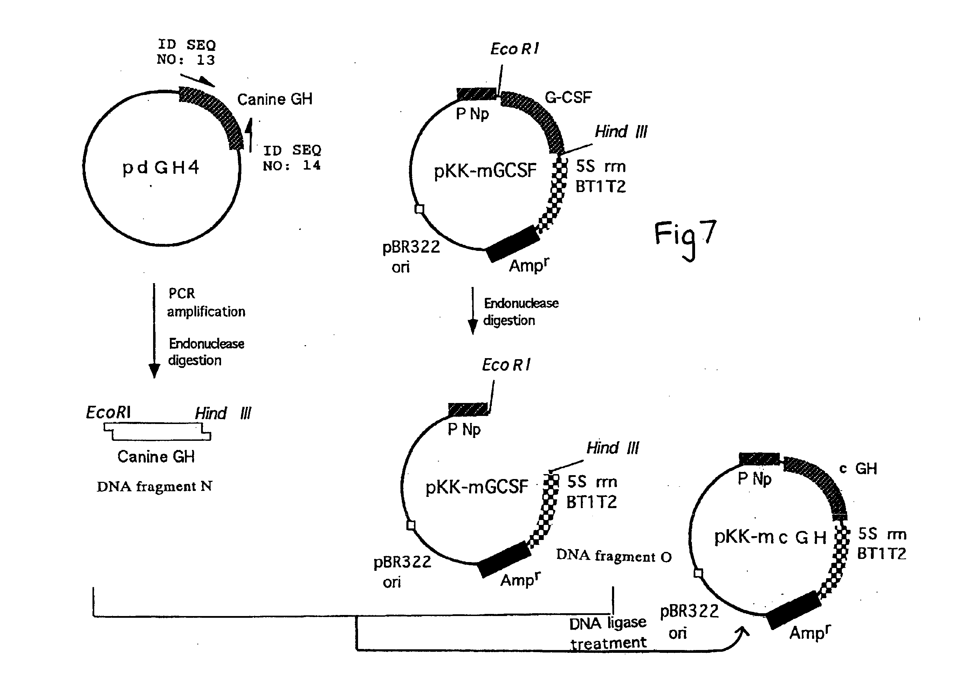

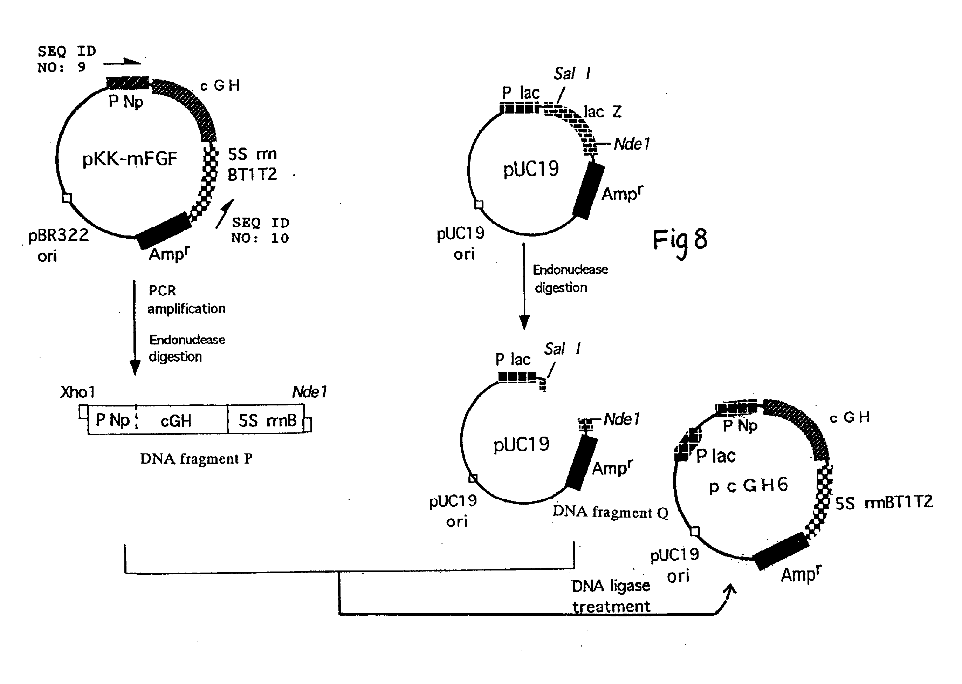

There will be described this invention with reference to, but not limited to, Examples and Comparative Examples. In the following description, "%" is based on a weight, unless otherwise indicated. Construction processes for individual plasmids below are shown in Figs. 1 to 8.

### Example 1: Application of a DNA cassette to hG-CSF production

### (1) Construction of an hG-CSF producing strain

### i. Construction of a cDNA library

mRNA was extracted from A-431 cultured cells derived from human vulvar squamous cell carcinoma (ATCC CRL-1555) by guanidine thiocyanate-cesium chloride technique and then Quick Prep polyA(+) RNA Purification Kit (Pharmacia) was used to prepare polyA(+) RNA. In addition, ZAP-cDNA was prepared from the m-RNA and cDNA was prepared from Synthesis Kit (STRATAGENE).

### ii. Preparation of 5'-end modified hG-CGF gene DNA fragment A

Using oligonucleotides having the sequences of SEQ ID NOs: 5 and 6, respectively, as a primer, PCR was conducted to give a DNA fragment comprising an hG-CSF gene from the cDNA library in which the 5'-end sequence was modified to be enriched with adenine and thymine. The 5'-end sequences before and after modification are shown below.

### Before modification

ATG ACT CCA TTA GGT CCT GCT TCT TCT CTG CCG CAG

### After modification

ATG ACC CCC CTG GGC CCT GCC AGC TCC CTG CCC CAG

This DNA fragment was treated with restriction enzymes EcoRI and HindIII to provide DNA fragment A.

### iii. Preparation of DNA fragment B comprising an Np promoter

Using a plasmid (pGHR10) extracted from cultured cells of MT-10765 strain (FERM BP-5020) as a template and oligonucleotides having the sequences of SEQ ID NOs: 7 and 8, respectively, as a primer, PCR was conducted to give a DNA fragment comprising an Np promoter. This DNA fragment was treated with restriction enzymes (endonuclease) PuvII and EcoRI to provide DNA fragment B.

### iv. Preparation of an Np promoter, an hG-CSF gene and a plasmid comprising a terminator region (pKK-mGCSF)

Commercially available pKK223-3 (Pharmacia) for expression was digested with restriction enzymes HindIII and PvuII. A longer fragment, i.e., DNA fragment C with about 2.6 kbp was collected by agarose gel electrophoresis. DNA fragment C was ligated with the above DNA fragments A and B using a commercially available ligation kit (Takara Shuzo). A reaction liquid containing the conjugate of these DNA fragments was used to transform a E. coli DH5α strain competent cell (Toyobo). Then, the resulting E. coli transformant was used to prepare an hG-CSF expression plasmid (pKK-mGCSF) comprising the Np promoter.

### v. Preparation of a gene fragment comprising an Np promoter, an hG-CSF gene and a terminator region

Using the above pKK-mGCSF as a template and oligonucleotides having the sequences of SEQ ID NOs: 9 and 10, respectively, as a primer, PCR was conducted to give a DNA fragment comprising the Np promoter, the hG-CSF gene and a 5SrrBT1T2 terminator region. The DNA fragment was treated with restriction enzymes XhoI and NdeI to provide DNA fragment D.

### vi. Construction of an hG-CSF expression plasmid comprising a cassette for expression (pGCSF6)

A commercially available expression vector pUC19 (New England Lab.) was digested with restriction enzymes SalI and NdeI. A longer fragment, i.e., DNA fragment E with about 2.4 kbp was collected by agarose gel electrophoresis. DNA fragment E was ligated with the above DNA fragment D to construct an hG-CSF expression plasmid comprising a lac-Np tandem promoter (DNA cassette) (pGCSF6). The cassette for expression had a sequence of SEQ ID NO: 4.

### vii. Construction of an hG-CSF expressing E. coli strain

Competent cells were prepared from cultured cells of E. coli W3110 strain (ATCC 27325) by common calcium chloride technique. These were transformed with the above plasmid (pGCSF6) to construct an hG-CSF expressing E. coli strain W3110/pGCSF6.

### (2) Culturing an E. coli strain producing an hG-CSF

The W3110/pGCSF6 strain was inoculated into a 2× LB medium (20 g/L polypeptone and 10 g/L yeast extract) containing 100 µg/L and 0.75 % glycelol. The medium was cultured with stirring while being aerated at 37 °C for 16 hours to allow hG-CSF to be accumulated in cells as an inclusion body.

### (3) Estimation of the amount of expressed hG-CSF

After culturing in (2), cultured cells were collected from the culture medium as a precipitant by centrifugation. A proportion of hG-CSF as protein in the total protein weight in the cultured cells was assayed from a chromatic figure with Coomassie Blue dye after electrophoresis with an SDS polyacrylamide gel (Daiichi Chemical, Multigel 15/25). The results demonstrated that 50 % or more of the total proteins in the cells was hG-CSF.

### (4) Purification of hG-CSF

The cells were collected from the culture medium obtained in (2) by centrifugation. After disruption of the cells with a French press, the debris was repeatedly washed by centrifugation to prepare inclusion bodies containing hG-CSF of 95 % or higher protein purity. The inclusion bodies were subject to unfolding/refolding as described in JP-A 2-234692 to give activated hG-CSF. After subsequent column purification, hG-CSF purified to a single band as determined by electrophoresis analysis using the SDS polyacrylamide gel was obtained.

### (5) Determining biological activity of purified hG-CSF using NFS-60

The purified hG-CSF obtained as described above was determined for its biological activity as follows. NFS-60 cells (Proc. Natl. Acad. Sci., USA, 82, 6687-6691, 1985; J. Pharm. Biomed. Anal., 13, 9-20, 1995) were inoculated at 1 × 10⁴ cells/mL into a cell subculture medium prepared by adding 15 % fetal bovine serum (PAA Laboratories), 100 µM 2-mercaptoethanol (SIGMA) and 10 ng/mL murine IL-3 (R&D systems) to Isocove's Modified Dulbecco's Medium (hereinafter, referred to as "IMDM": GIBCO), and were then pre-cultured for 3 days. Then, the subcultured NFS-60G cells were centrifuged at 1000 rpm for 5 min and the supernatant was removed. The cells were washed with PBS(-) (Nissui Pharm.) and again centrifuged, and then the supernatant was removed. The cells were suspended at 5 × 10⁵ cells/mL in a cell subculture medium without murine IL-3, and then cultured for 16 hours under the conditions of 37 °C, 5 % CO₂ and 100 % relative humidity. After preculturing, the cells in a cell subculture medium without murine IL-3 were inoculated in a 96 well plate for tissue culture (IWAKI Glass) using a microdispensor at 2 × 10³ cells / 50 µL·well. Then, the purified hG-CSF was added at 50 µL/well. After culturing under the conditions of 37 °C, 5 % CO₂ and 100 % relative humidity for 2 days, a Tetra Color One (Biochemical Industries) solution was added at 10 µL/well and culturing was continued for additional 4 hours. After culturing, the plate was shaken by a plate shaker for 1 min and an absorbance at 450 nm (control: absorbance at 595 nm) was determined to detect the degree of cell growth. Using an hG-CSF preparation (Glan injectable liquid, Sankyo) , the same procedure was conducted. The results demonstrated that the hG-CSF obtained from E. coli as described above has activity per a unit hG-CSF protein corresponding to a commercially available product.

### Comparative Example 1

### (1) Construction of an hG-CSF expression plasmid comprising a lac promoter (pLAC-mGCSF)

### i. Construction of an hG-CSF expression plasmid comprising a lac promoter (pLAC-mGCSF)

Using the hG-CSF expression plasmid comprising the Np promoter and the terminator region (pKK-mGCSF) as a template and oligonucleotides having the sequences of SEQ ID NOs: 5 and 10, respectively, as a primer, PCR was conducted to give a DNA fragment comprising a 5'-end modified hG-CSF gene and the terminator region. The DNA fragment was digested with restriction enzymes EcoRI and NdeI and then subject to agarose gel electrophoresis to collect a shorter fragment, i.e., DNA fragment F with about 900 bp. The DNA fragment F comprising the hG-CSF gene and the terminator region was ligated to DNA fragment G prepared by digesting the expression vector pUC19 with the above restriction enzymes EcoRI and NedI to construct a plasmid comprising only a lac promoter (pLAC-mGCSF) for the hG-CSF gene expression.

### ii. Construction of an hG-CSF expressing E. coli strain

Competent cells were prepared from cultured cells of E. coli W3110 strain (ATCC 27325) by common calcium chloride technique. These were transformed with the above plasmid (pLAC-mGCSF) to construct an hG-CSF expressing E. coli strain W3110/pLAC-mGCSF.

### (2) Construction of a plasmid for hG-CSF production comprising an Np promoter

### i. Preparation of a DNA fragment comprising an Np promoter, an hG-CSF gene and a terminator region

Using the above pKK-mGCSF as a template and oligonucleotides having the sequences of SEQ ID NOs: 9 and 10, respectively, as a primer, PCR was conducted to give a DNA fragment comprising the Np promoter, the hG-CSF gene and a terminator region. The DNA fragment was treated with a restriction enzyme NdeI to provide DNA fragment H.

### ii. Construction of an hG-CSF expression plasmid comprising an Np promoter(pNPR-mGCSF)

A expression vector pUC19 was digested with restriction enzymes PvuII and NdeI. A longer fragment, i.e., DNA fragment I with about 2.2 kbp was collected by agarose gel electrophoresis. DNA fragment I was ligated with the above DNA fragment H to construct an hG-CSF expression plasmid comprising only the Np promoter for hG-CSF expression (pNpr-mGCSF).

### iii. Construction of an hG-CSF expressing E. coli strain

Competent cells were prepared from cultured cells of E. coli W3110 strain (ATCC 27325) by common calcium chloride technique. These were transformed with the above plasmid (pNpr-mGCSF) to construct an hG-CSF expressing E. coli strain W3110/pNpr-mGCSF.

### (3) Comparison of hG-CSF expression amounts for using individual promoters by culturing

### i. Comparison of hG-CSF expression amounts when the lac and the Np promoters are separately used (W3110/pLAC-mGCSF and W3110/pNpr-mGCSF strains)

The W3110/pLAC-mGCSF strain was cultured overnight in an LB medium (10 g/L polypeptone and 5 g/L yeast extract) containing 100 µg/L ampicillin at 37 °C. Next day, 5 % volume was inoculated into a similar medium. When the turbidity (OD₆₀₀) based on suspended cells reached about 2, an inducer, IPTG, was added to 5 mM and culturing was continued for further 3 hours.

The W3110/pNpr-mGCSF strain was cultured as described in Example 1 for the W3110/pGCSF6.

Cells were collected from the media of these two strains, respectively. These were analyzed for an hG-CSF amount per a unit protein in the E. coli cells by SDS polyacrylamide gel electrophoresis and the results were compared. Thus, an hG-CSF content was about 10 % in all strains, which was about 1/5 of that for the W3110/pGSCF6 strain described in Example 1.

### Example 2

### (1) Construction of an hFGF-2 producing strain comprising a cassette for protein expression

### (1-1) Preparation of an hFGF-2 gene (DNA fragment J)

Using oligonucleotides having the sequences of SEQ ID NOs: 11 and 12, respectively, as a primer, PCR was conducted to give a DNA fragment comprising an hFGF-2 gene from the cDNA library as described in Example 1 in which the 5'-end sequence was modified to be enriched with adenine and thymine. This DNA fragment was treated with restriction enzymes EcoRI and HindIII.

### (1-2) Preparation of a plasmid comprising an Np promoter (pKK-mFGF)

The hG-CSF expression plasmid comprising Np promoter described in Example 1 (pKK-mGCSF) was digested with restriction enzymes EcoRI and HindIII. A longer fragment, i.e., DNA fragment K with about 2.7 kbp was collected by agarose gel electrophoresis. DNA fragment K was ligated with the above DNA fragment J to construct an hFGF-2 expression plasmid comprising the Np promoter (pKK-mFGF).

### (1-3) Preparation of a DNA fragment comprising an Np promoter, an hFGF-2 gene and a terminator region

Using the above pKK-mFGF as a template and oligonucleotides having the sequences of SEQ ID NOs: 9 and 10, respectively, as a primer, PCR was conducted to give a DNA fragment comprising the Np promoter, the 5'-end modified hFGF-2 gene and a 5SrrBT1T2 terminator region. The DNA fragment was treated with restriction enzymes XhoI and NdeI to provide DNA fragment L.

### (1-4) Construction of an hFGF-2 expression plasmid comprising a cassette for expression (pFGF6)

A commercially available expression vector pUC19 (New England Lab.) was digested with restriction enzymes SalI and NdeI. A longer fragment, i.e., DNA fragment M with about 2.4 kbp was collected by agarose gel electrophoresis. The fragment was ligated with the above DNA fragment L to construct an hFGF-2 expression plasmid comprising a DNA cassette for protein expression (pFGF6).

### (1-5) Construction of an hFGF-2 expressing E. coli strain

Competent cells were prepared from cultured cells of E. coli W3110 strain (ATCC 27325) by common calcium chloride technique. These were transformed with the above plasmid (pFGF6) to construct an hFGF-2 expressing E. coli strain W3110/pFGF6.

### (2) Culturing an hFGF-2 expressing E. coli strain

The W3110/pFGF6 strain was cultured as described in Example 1 for the hG-CSF expressing E. coli strain to prepare E. coli cells within which a desired recombinant protein was stored as a soluble protein.

### (3) Estimation of the amount of expressed hFGF-2

Cultured cells were collected from the culture medium obtained in (2) by centrifugation. A content of hFGF-2 per a unit protein in the cells was assayed by SDS polyacrylamide gel electrophoresis. The results demonstrated that 45 % or more of the total proteins in the W3110/pFGF6 cultured cells was hFGF-2.

### (4) Determining biological activity of an hFGF-2 producing cell extract

Biological activity of an extract of the disrupted hFGF-2 producing E. coli strain collected from the culture medium obtained from (2) by centrifugation was determined according to the determination process for biological activity using murine BALB/c3T3 cells disclosed in JP-B 8-2526965. The results demonstrated that the hFGF-2 recovered into the disrupted cell extract had a given level of activity.

### Example 3

### (1) Construction of a canine growth hormone producing strain comprising a cassette for protein expression

### (1-1) Preparation of a plasmid comprising a canine growth hormone gene (DNA fragment N)

Using a plasmid pdGH4 comprising a canine growth hormone (Mie Medical Journal, 44, 125-132 (1994)) as a template and oligonucleotides having the sequences of SEQ ID NOs: 13 and 14, respectively, as a primer, PCR was conducted to give a DNA fragment comprising a canine growth hormone gene in which the 5'-end sequence was modified to be enriched with adenine and thymine. This DNA fragment was treated with restriction enzymes EcoRI and HindIII to give DNA fragment N.

### (1-2) Preparation of a plasmid comprising an Np promoter and a terminator region (pKK-mcGH)

The hG-CSF expression plasmid comprising Np promoter described in Example 1 (pKK-mGCSF) was digested with restriction enzymes EcoRI and HindIII. A longer fragment, i.e., DNA fragment O with about 2.7 kbp was collected by agarose gel electrophoresis. DNA fragment O was ligated with the above DNA fragment N to construct a canine growth hormone expression plasmid comprising the Np promoter (pKK-mcGH).

### (1-3) Preparation of a DNA fragment comprising an Np promoter, a canine growth hormone gene and a terminator region

Using the above pKK-mcGH as a template and oligonucleotides having the sequences of SEQ ID NOs: 9 and 10, respectively, as a primer, PCR was conducted to give a DNA fragment comprising the Np promoter, the modified canine growth hormone gene and a 5SrrBT1T2 terminator region. The DNA fragment was treated with restriction enzymes XhoI and NdeI to provide DNA fragment P.

### (1-4) Construction of a canine growth hormone expression plasmid comprising a cassette for expression (pCGH6)

A commercially available pUC19 plasmid was digested with restriction enzymes SalI and NdeI. A longer fragment, i.e., DNA fragment Q with about 2.4 kbp was collected by agarose gel electrophoresis. DNA fragment Q was ligated with the above DNA fragment P to construct a canine growth hormone expression plasmid comprising a cassette for protein expression (pCGH6).

### (1-5) Construction of a canine growth hormone expressing E. coli strain

Competent cells were prepared from cultured cells of E. coli W3110 strain (ATCC 27325) by common calcium chloride technique. These were transformed with the above plasmid (pCGH6) to construct a canine growth hormone expressing E. coli strain W3110/pCGH6.

### (2) Culturing a canine growth hormone expressing E. coli strain

The W3110/pCGH6 strain was cultured as described in Example 1 for the hG-CSF expressing E. coli strain to prepare E. coli cells within which a desired recombinant protein was stored as a soluble protein.

### (3) Estimation of the amount of expressed canine growth hormone

Cultured cells were collected from the culture medium obtained in (2) by centrifugation. A content of canine growth hormone per a unit protein in the cells was assayed by SDS polyacrylamide gel electrophoresis. The results demonstrated that 60 % or more of the total proteins in the W3110/pCGH6 strain was canine growth hormone.

### (4) Purification of canine growth hormone

Inclusion bodies of canine growth hormone were collected from the disrupted cell liquid prepared by disrupting the cultured cells by centrifugation. These were repeatedly subject to unfolding/refolding, and purified as described in Biochemistry, 34, 5773-5794 (1995) to prepare purified canine growth hormone to a single band as determined by SDS polyacrylamide gel electrophoresis.

### (5) Determining biological activity of the purified canine growth hormone using a rat without a pituitary gland

It was demonstrated that the purified canine growth hormone had activity according to a growth activity determination test using a rat without a pituitary gland (Endocrinology, Vol. 120, No. 6, pp. 2582-2590 (1987)).

As described above, this invention allows us to produce a protein derived from a mammal, in particular human granulocyte colony stimulating factor (hG-CSF), human fibroblast growth factor (hFGF-2) or canine growth hormone in a higher production amount which cannot be achieved using E. coli as a host according to the prior art.

The sequences with SEQ ID Nos have the following concrete sequences, respectively:

Sequence Listing
<110> MITSUI CHEMICALS, INC.
<120> A Method of Production of A Protein
<160> 14
<210>1
<211> 213
<212> DNA
<213> Artificial Sequence
<220> Promoter
<400> 1
gcccaatacg caaaccgcct ctccccgcgc gttggccgat tcattaatgc agctggcacg 60
acaggtttcc cgactggaaa gcgggcagtg agcgcaacgc aattaatgtg agttagctca 120
ctcattaggc accccaggct ttacacttta tgcttccggc tcgtatgttg tgtggaattg 180
tgagcggata acaatttcac acaggaaaca gct 213
<210> 2
<211> 84
<212> DNA
<213> Artificial Sequence
<220> Promoter
<400> 2
gcggagtcta gttttatatt gcagaatgcg agattgctgg tttattataa caatataagt 60
tttcattatt ttcaaaaagg ggat 84
<210> 3
<211> 144
<212> DNA
<213> Artificial Sequence
<220> Promoter
<400> 3
cggcttatcc cctgacaccg cccgccgaca gcccgcatgg acgaatctat caattcagcc 60
gcggagtcta gttttatatt gcagaatgcg agattgctgg tttattataa caatataagt 120
tttcattatt ttcaaaaagg ggat 144
<210> 4
<211> 337
<212> DNA
<213> Artificial Sequence
<220> Promoter

<400> 4
gcccaatacg caaaccgcct ctccccgcgc gttggccgat tcattaatgc agctggcacg 60
acaggtttcc cgactggaaa gcgggcagtg agcgcaacgc aattaatgtg agttagctca 120
ctcattaggc accccaggct ttacacttta tgcttccggc tcgtatgttg tgtggaattg 180
tgagcggata acaatttcac acaggaaaca gctatgacca tgattacgcc aagcttgcat 240
gcctgcaggt cgagcggagt ctagttttat attgcagaat gcgagattgc tggtttatta 300
tacaatataa gttttcatta ttttcaaaaa gggggat 337
<210> 5
<211> 45
<212> DNA
<213> Artificial Sequence
<220> Primer
<400> 5
acagaattca tgactccatt aggtcctgct tcttctctgc cgcag 45
<210> 6
<211> 27
<212> DNA
<213> Artificial Sequence
<220> Primer
<400> 6
gggaagcttt cagggctggg caaggtg
<210> 7
<211> 28
<212> DNA
<213> Artificial Sequence
<220> Primer
<400> 7
tttcagctgt gcaactttat ccgcctcc 28
<210> 8
<211> 31
<212> DNA
<213> Artificial Sequence
<220> Primer
<400> 8
catgaattca accccctttt tgaaaataat g
<210> 9

<211> 49
<212> DNA
<213> Artificial Sequence
<220> Primer
<400> 9
tttcatatgc agctgctcga gcggagtcta gttttatatt gcagaatgc 49
<210> 10
<211> 27
<212> DNA
<213> Artificial Sequence
<220> Primer
<400> 10
aaacatatgg agtttgtaga aacgcaa 27
<210> 11
<211> 55
<212> DNA
<213> Artificial Sequence
<220> Primer
<400> 11
tctagaattc atggctgctg gttctatcac tactctgccc gccttgcccg aggat 55
<210> 12
<211> 30
<212> DNA
<213> Artificial Sequence
<220> Primer
<400> 12
tactaagctt ggccattaaa atcagctctt 30
<210> 13
<211> 60
<212> DNA
<213> Artificial Sequence
<220> Primer
<400> 13
atagaattca tgttcccagc tatgccatta tcttctttat ttgctaacgc cgtgctccgg 60
<210> 14
<211> 31

<212> DNA
<213> Artificial Sequence
<220> Primer
<400> 14
attaagcttc tagaaggcac agctgctttc c 31

SEQ ID NO: 1

gcccaatacg caaaccgcct ctccccgcgc gttggccgat tcattaatgc

agctggcacg acaggtttcc cgactggaaa gcgggcagtg agcgcaacgc

aattaatgtg agttagctca ctcattaggc accccaggct ttacacttta

tgcttccggc tcgtatgttg tgtggaattg tgagcggata acaatttcac

acaggaaaca gct

SEQ ID NO:2

gcggagtcta gttttatatt gcagaatgcg agattgctgg tttattataa

caatataagt tttcattatt ttcaaaaagg ggat

SEQ ID NO: 3

cggcttatcc cctgacaccg cccgccgaca gcccgcatgg acgaatctat

caattcagcc gcggagtcta gttttatatt gcagaatgcg agattgctgg

tttattataa caatataagt tttcattatt ttcaaaaagg ggat

SEQ ID NO: 4

gcccaatacg caaaccgcct ctccccgcgc gttggccgat tcattaatgc

agctggcacg acaggtttcc cgactggaaa gcgggcagtg agcgcaacgc

aattaatgtg agttagctca ctcattaggc accccaggct ttacacttta

tgcttccggc tcgtatgttg tgtggaattg tgagcggata acaatttcac
acaggaaaca gctatgacca tgattacgcc aagcttgcat gcctgcaggt
cgagcggagt ctagttttat attgcagaat gcgagattgc tggtttatta
tacaatataa gttttcatta ttttcaaaaa gggggat
SEQ ID NO: 5
acagaattca tgactccatt aggtcctgct tcttctctgc cgcag
SEQ ID NO: 6
gggaagcttt cagggctggg caaggtg
SEQ ID NO: 7
tttcagctgt gcaactttat ccgcctcc
SEQ ID NO: 8
catgaattca accccctttt tgaaaataat g
SEQ ID NO: 9
tttcatatgc agctgctcga gcggagtcta gttttatatt gcagaatgc
SEQ ID NO: 10
aaacatatgg agtttgtaga aacgcaa
SEQ ID NO: 11
tctagaattc atggctgctg gttctatcac tactctgccc gccttgcccg
aggat
SEQ ID NO: 12
tactaagctt ggccattaaa atcagctctt
SEQ ID NO: 13
atagaattca tgttcccagc tatgccatta tcttctttat ttgctaacgc
cgtgctccgg
SEQ ID NO: 14
attaagcttc tagaaggcac agctgctttc c

### Annex to the application documents-subsequently filed sequence listing

SEQUENCE LISTING

<110> Mitsui Chemicals, Inc.

<120> Process for producing a protein

<130> IS/FP5960083

<140> EP 01308502.2
<141> 2001-10-04

<150> JP 2000-305132
<151> 2000-10-04

<160> 16

<170> PatentIn Ver. 2.1

<210> 1
<211> 213
<212> DNA
<213> Artificial Sequence

<220>
<223> Description of Artificial Sequence: Promoter

<400> 1
gcccaatacg caaaccgcct ctccccgcgc gttggccgat tcattaatgc agctggcacg 60
acaggtttcc cgactggaaa gcgggcagtg agcgcaacgc aattaatgtg agttagctca 120
ctcattaggc accccaggct ttacacttta tgcttccggc tcgtatgttg tgtggaattg 180
tgagcggata acaatttcac acaggaaaca gct 213

<210> 2

<211> 84

<212> DNA

<213> Artificial Sequence

<220>

<223> Description of Artificial Sequence: Promoter

<400> 2

gcggagtcta gttttatatt gcagaatgcg agattgctgg tttattataa caatataagt 60

tttcattatt ttcaaaaagg ggat 84

<210> 3

<211> 144

<212> DNA

<213> Artificial Sequence

<220>

<223> Description of Artificial Sequence: Promoter

<400> 3

cggcttatcc cctgacaccg cccgccgaca gcccgcatgg acgaatctat caattcagcc 60

gcggagtcta gttttatatt gcagaatgcg agattgctgg tttattataa caatataagt 120

tttcattatt ttcaaaaagg ggat 144

<210> 4

<211> 337

<212> DNA

<213> Artificial Sequence

<220>

<223> Description of Artificial Sequence: Promoter

<400> 4

gcccaatacg caaaccgcct ctccccgcgc gttggccgat tcattaatgc agctggcacg 60

acaggtttcc cgactggaaa gcgggcagtg agcgcaacgc aattaatgtg agttagctca 120

ctcattaggc accccaggct ttacacttta tgcttccggc tcgtatgttg tgtggaattg 180
tgagcggata acaatttcac acaggaaaca gctatgacca tgattacgcc aagcttgcat 240
gcctgcaggt cgagcggagt ctagttttat attgcagaat gcgagattgc tggtttatta 300
tacaatataa gttttcatta ttttcaaaaa gggggat 337

<210> 5
<211> 45
<212> DNA
<213> Artificial Sequence

<220>
<223> Description of Artificial Sequence: Primer

<400> 5
acagaattca tgactccatt aggtcctgct tcttctctgc cgcag 45

<210> 6
<211> 27
<212> DNA
<213> Artificial Sequence

<220>
<223> Description of Artificial Sequence: Primer

<400> 6
gggaagcttt cagggctggg caaggtg 27

<210> 7
<211> 28
<212> DNA
<213> Artificial Sequence

<220>
<223> Description of Artificial Sequence: Primer

<400> 7
tttcagctgt gcaactttat ccgcctcc                                          28

<210> 8
<211> 31
<212> DNA
<213> Artificial Sequence

<220>
<223> Description of Artificial Sequence: Primer

<400> 8
catgaattca accccctttt tgaaaataat g                                      31

<210> 9
<211> 49
<212> DNA
<213> Artificial Sequence

<220>
<223> Description of Artificial Sequence: Primer

<400> 9
tttcatatgc agctgctcga gcggagtcta gttttatatt gcagaatgc                   49

<210> 10
<211> 27
<212> DNA
<213> Artificial Sequence

<220>
<223> Description of Artificial Sequence: Primer

<400> 10
aaacatatgg agtttgtaga aacgcaa                                           27

<210> 11
<211> 55
<212> DNA
<213> Artificial Sequence

<220>
<223> Description of Artificial Sequence: Primer

<400> 11
tctagaattc atggctgctg gttctatcac tactctgccc gccttgcccg aggat 55

<210> 12
<211> 30
<212> DNA
<213> Artificial Sequence

<220>
<223> Description of Artificial Sequence: Primer

<400> 12
tactaagctt ggccattaaa atcagctctt 30

<210> 13
<211> 60
<212> DNA
<213> Artificial Sequence

<220>
<223> Description of Artificial Sequence: Primer

<400> 13
atagaattca tgttcccagc tatgccatta tcttctttat ttgctaacgc cgtgctccgg 60

<210> 14
<211> 31
<212> DNA
<213> Artificial Sequence

<220>
<223> Description of Artificial Sequence: Primer

<400> 14
attaagcttc tagaaggcac agctgctttc c                                31

<210> 15
<211> 36
<212> DNA
<213> Homo sapiens

<400> 15
atgactccat taggtcctgc ttcttctctg ccgcag                           36

<210> 16
<211> 36
<212> DNA
<213> Artificial Sequence

<220>
<223> Description of Artificial Sequence: 5'-end
      modified DNA fragment

<400> 16
atgacccccc tgggccctgc cagctccctg ccccag                           36

## Claims

1. A DNA cassette functioning as a promoter for protein expression in E. coli, comprising the first promoter derived from a lac promoter region in a pUC 19 plasmid, a spacer region and the second promoter derived from a promoter region in a neutral protease in Bacillus amyloliquefaciens wherein the first promoter, the spacer region and the second promoter are sequentially aligned in series in a 5' to 3' direction.

2. The DNA cassette as claimed in Claim 1 wherein the first promoter has a sequence of SEQ ID NO: 1.

3. , The DNA cassette as claimed in Claim 1 or 2 wherein the spacer region is a DNA of 25 to 45 bp.

4. The DNA cassette as claimed in Claim 3 wherein the spacer region consists of a DNA of 25 to 45 bp from the 5' end of a lacZ gene in the pUC19 plasmid.

5. The DNA cassette as claimed in any one of Claims 1 to 4 wherein the second promoter has a sequence of SEQ ID NO. 2.

6. The DNA cassette as claimed in any one of Claims 1 to 4 wherein the second promoter has a sequence of SEQ ID NO. 3.

7. The DNA cassette as claimed in Claim 1 having a sequence of SEQ ID NO. 4.

8. A plasmid comprising the DNA cassette as claimed in any one of Claims 1 to 7 and a region permitting replication in E. coli.

9. An E. coli comprising the plasmid as claimed in Claim 8.

10. A recombinant plasmid for protein expression in E. coli comprising the DNA cassette as claimed in any one of Claims 1 to 7; a coding region encoding a protein which is functionally ligated to the 3' end in the DNA cassette; and a region for replication in E. coli.

11. The recombinant plasmid as claimed in Claim 10 wherein the protein is derived from a mammal.

12. The recombinant plasmid as claimed in Claim 11 wherein the protein is selected from the group consisting of human granulocyte colony stimulating factor (hG-CSF), human fibroblast growth factor (hFGF-2) and canine growth hormone.

13. A recombinant E. coli which is transformed with the recombinant plasmid as claimed in any of Claims 10 to 12 and can produce the protein encoded in the coding region.

14. A process for producing a protein comprising the steps of culturing a recombinant E. coli as claimed in Claim 13 to produce the protein encoded in the coding region in the recombinant plasmid,carried by the recombinant E. coli within the cells of the E. coli; and recovering the protein from the cells.
